# EUROPÄISCHE PATENTSCHRIFT

(11) **EP 1 776 087 B1**
(45) Veröffentlichungstag und Bekanntmachung des Hinweises auf die Patenterteilung: **25.06.2014**
(21) Anmeldenummer: 05770877.8
(22) Anmeldetag: 10.08.2005
(51) Int. Cl.: A61Q 19/00, A61Q 19/08, A61K 8/64, A61K 8/42

(54) **KOSMETISCHE UND DERMATOLOGISCHE ZUSAMMENSETZUNGEN MIT (2-HYDROXYETHYL)HARNSTOFF**
COSMETIC AND DERMATOLOGICAL COMPOSITIONS COMPRISING (2-HYDROXYETHYL)UREA
COMPOSITIONS COSMETIQUES ET DERMATOLOGIQUES A BASE DE (2-HYDROXYETHYL)UREE

(30) Priorität: 13.08.2004 DE 102004039550; 15.10.2004 DE 102004050563; 17.11.2004 DE 102004055541; 05.01.2005 DE 102005000868; 07.06.2005 DE 102005026357
(43) Veröffentlichungstag der Anmeldung: 25.04.2007
(60) Teilanmeldung: 10182140.3
(73) Patentinhaber: Henkel AG & Co. KGaA, 40589 Düsseldorf (DE)
(72) Erfinder: DÖRING, Thomas, 41542 Dormagen (DE); TRÄGER, Anemone, 40227 Düsseldorf (DE); WALDMANN-LAUE, Marianne, 40789 Monheim (DE); ANDERHEGGEN, Bernd, 42781 Haan (DE)
(86) Internationale Anmeldenummer: PCT/EP2005/008661
(87) Internationale Veröffentlichungsnummer: WO 2006/018198

(56) Entgegenhaltungen:
- EP-A- 1 535 607
- WO-A-02/49593
- WO-A-89/12441
- DE-A1- 2 703 185
- FR-A- 2 740 453
- FR-A- 2 746 316
- US-A1- 2003 204 061
- US-A1- 2004 057 917

## Beschreibung

Die Erfindung betrifft topische kosmetische oder dermatologische Zusammensetzungen, die in einem geeigneten kosmetischen oder dermatologischen Träger eine Kombination von (2-Hydroxyethyl) - harnstoff mit Sojaproteinhydrolysaten.

Topische Zusammensetzungen, die kosmetische oder dermatologische Wirkstoffe enthalten, sind im Stand der Technik bekannt. Ein häufig auftretender Nachteil ist allerdings die begrenzte Wirksamkeit, da die Wirkstoffe meist an der obersten Schicht der Epidermis verbleiben, deren Zellen bereits die letzte Stufe vor der Desquamation im Kreislauf der ständigen Erneuerung der Epidermis darstellen. Wünschenswert dagegen wäre zum Beispiel die Einschleusung der applizierten Wirkstoffe auch in tiefere Schichten der Epidermis, damit die Wirkstoffe der Haut insgesamt länger zur Verfügung stehen, um ihre maximale Wirkung erzielen zu können. Da die kosmetischen oder dermatologischen Wirkstoffe für einen beträchtlichen Anteil der Rohstoffkosten einer topischen Zusammensetzung verantwortlich sind, ist eine verbesserte Ausnutzung der Wirkstoffe, bevorzugt unter Ausnutzung von Synergieeffekten, von großem ökonomischen Interesse für den Hersteller.

Aufgabe der vorliegenden Erfindung war es, topische kosmetische oder dermatologische Zusammensetzungen mit einer optimierten Wirksamkeit der enthaltenen Wirkstoffe bereitzustellen.

Eine weitere Aufgabe der vorliegenden Erfindung war es, topische kosmetische oder dermatologische Zusammensetzungen zur nicht-therapeutischen, kosmetischen Behandlung und/oder Minimierung von trockener Haut bereitzustellen.

Alkyl- oder hydroxyalkylsubstituierte Harnstoffe, insbesondere (2-Hydroxyethyl)harnstoff, sind, ähnlich wie Harnstoff selbst, im Stand der Technik als feuchtigkeitsspendende und hautweichmachende Wirkstoff bekannt, beispielsweise aus DE 2703185 A1.

Überraschend und für den Fachmann nicht vorhersehbar wurde nun gefunden, dass alkyl- oder hydroxyalkylsubstituierte Harnstoffe, insbesondere (2-Hydroxyethyl)-harnstoff, auch in der Lage sein können, die Wirksamkeit ausgewählter kosmetischer oder dermatologischer Wirkstoffe zu optimieren.

Der Gegenstand der vorliegenden Erfindung ist in den Ansprüchen definiert.

Erfindungsgemäß besonders bevorzugte Zusammensetzungen sind dadurch gekennzeichnet, dass sie (2-Hydroxyethyl)harnstoff in einer Gesamtmenge von 0,01 - 50 Gew.-%, bevorzugt 0,1 - 20 Gew.-%, besonders bevorzugt 1 - 10 Gew.-% und außerordentlich bevorzugt 2 - 5 Gew.-%, bezogen auf die gesamte Zusammensetzung, enthalten. (2-Hydroxyethyl)harnstoff, bei Raumtemperatur ein kristalliner Feststoff, ist beispielsweise als Handelsprodukt Hydrovance der National Starch and Chemical Company als ca. 45 - 55 %ige wässrige Lösung erhältlich.

Besonders bevorzugt sind Sojaproteinhydrolysate mit einem mittleren Molekulargewicht im Bereich von 1200 - 1800 Dalton, bevorzugt im Bereich von 1400 - 1700 Dalton, z. B. unter dem Handelsnamen Ridulisse C^{®} von der Firma Silab erhältlich, und Sojaproteinhydrolysate mit einem mittleren Molekulargewicht im Bereich von 600 - 1000 Dalton, bevorzugt 800 Dalton, z. B. unter dem Handelsnamen Phytokine^{®} von Coletica erhältlich. mit Kokosfettsäuren N-acylierten und/oder veresterten Sojaproteinhydrolysaten in Form ihrer Alkalimetallsalze. Kokosfettsäuren umfassen überwiegend Alkancarbonsäuren mit einer Anzahl an Kohlenstoffatomen von 8 - 18, insbesondere Caprylsäure, Caprinsäure, Laurinsäure, Myristinsäure, Palmitinsäure und Stearinsäure. Bevorzugte Alkalimetallsalze sind ausgewählt aus Lithium-, Natrium- und Kaliumsalzen, wobei die Kaliumsalze besonders bevorzugt sind.

Ein weiteres, erfindungsgemäß besonders bevorzugtes Sojaproteinhydrolysat ist ein mit Kokosfettsäuren N-acyliertes und/oder verestertes Sojaproteinhydrolysat in Form des Kaliumsalzes, das unter der Handelsbezeichnung Coccopolipeptide di Soja von der Firma Sinerga erhältlich ist.

Vorteilhafterweise liegen die erfindungsgemäßen Hautbehandlungsmittel in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen klebstoff geeigneten Polymer vor. Die Mittel können auch in wasserfreie Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

In der Ausführungsform als Emulsion oder als tensidische Lösung, z. B. als Reinigungsmittel, enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®}68, Anlagerungs-produkte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsäuren und deren Na-, K-, Ammonium-, Ca-, Mg- und Zn - Salze.

Die erfindungsgemäßen Mittel enthalten die Emulgatoren bevorzugt in Mengen von 0,1 bis 25 Gew.-%, insbesondere 0,5 - 15 Gew.-%, bezogen auf das gesamte Mittel.

In einer besonders bevorzugten Ausführungsform ist mindestens ein nichtionischer Emulgator mit einem HLB-Wert von 8 und darunter enthalten. Derart geeignete Emulgatoren sind beispielsweise Verbindungen der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxygruppen ist. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäüre oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythrit-monoerucat.

Weitere geeignete Zusatzstoffe sind Fettstoffe, insbesondere pflanzliche Öle, wie Sonnenblumenöl, Olivenöl, Sojaöl, Rapsöl, Mandelöl, Jojobaöl, Orangenöl, Weizenkeimöl, Pfirsichkemöl und die flüssigen Anteile des Kokosöls, flüssige Paraffinöle, Isoparaffinöle und synthetische Kohlenwasserstoffe, Di-n-alkylether mit insgesamt 12 bis 36 C-Atomen, z. B. Di-n-octylether und n-Hexyl-n-octylether, Fettsäuren, besonders lineare und/oder verzweigte, gesättigte und/oder ungesättigte C₈₋₃₀-Fettsäuren, Fettalkohole, besonders gesättigte, ein- oder mehrfach ungesättigte, verzweigte oder unverzweigte Fettalkohole mit 6 - 30 Kohlenstoffatomen, Esteröle, das heißt Ester von C₆₋₃₀-Fettsäuren mit C₂₋₃₀-Fettalkoholen, Hydroxycarbonsäurealkylester, Dicarbonsäureester wie Di-n-butyladipat sowie Diolester wie Ethylenglykoldioleat oder Propylenglykoldi(2-ethylhexanoat), symmetrische, unsymmetrische oder cyclische Ester der Kohlensäure mit Fettalkoholen, z. B. Glycerincarbonat oder Dicaprylylcarbonat (Cetiol^{®} CC), Mono,-Di- und Trifettsäureester von gesättigten und/oder ungesättigten linearen und/oder verzweigten Fettsäuren mit Glycerin Wachse, insbesondere Insektenwachse, Pflanzenwachse, Fruchtwachse, Ozokerit, Mikrowachse, Ceresin, Paraffinwachse, Triglyceride gesättigter und gegebenenfalls hydroxylierter C₁₆₋₃₀-Fettsäuren, z. B. gehärtete Triglyceridfette, Siliconverbindungen, ausgewählt aus Decamethylcyclopentasiloxan, Dodecamethylcyclohexasiloxan und Siliconpolymeren, die gewünschtenfalls, quervernetzt sein können, z. B. Polydialkylsiloxane, Polyalkylarylsiloxane, ethoxylierte Polydialkylsiloxane sowie Polydialkylsiloxane, die Amin- und/oder Hydroxy-Gruppen enthalten.

Die bevorzugte Einsatzmenge der Fettstoffe beträgt 0.1 - 50 Gew.%, bevorzugt 0,1 - 20 Gew.% und besonders bevorzugt 0,1 - 15 Gew.%, jeweils bezogen auf die gesamte Zusammensetzung.

Weitere geeignete Zusatzstoffe sind Verdickungsmittel, z. B. natürliche und synthetische Tone und Schichtsilikate wie Bentonit, Hectorit, Montmorillonit oder Laponite^{®}, oder anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®}600, Simulgel^{®} NS und Simulgel^{®} EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich). Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.

Weitere geeignete Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum^{®}, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Trübungsmittel und Perlglanzmittel wie Ethylenglykolmono- und -distearat und Treibmittel wie Propan-Butan-Gemische, N₂O, Dimethylether, CO₂ und Luft.

Die nachfolgenden Beispiele sollen den Gegenstand der Erfindung erläutern, ohne ihn hierauf zu beschränken.

### 1. Oel-in-Wasser-Emulsionen nicht erfindungsgemäß

### 1. Beispielserie:

| | 1 | 2 | 3 |
|---|---|---|---|
| Distelöl | 3,00 | 3,00 | 3,00 |
| Myritol 318 | 5,00 | 5,00 | 5,00 |
| Novata AB | 2,00 | 2,00 | 2,00 |
| Behenyl Alcohol | 1,00 | 1,00 | 1,00 |
| Cutina MD | 2,00 | 2,00 | 2,00 |
| Lanette O | 1,00 | 1,00 | 1,00 |
| Isopropylstearat | 4,00 | 4,00 | 4,00 |
| Shea Butter | 2,00 | 2,00 | 2,00 |
| Dow Corning 200 Fluid 5 cst | 1,00 | 1,00 | 1,00 |
| Hydrogenated Palm Glycerides Citrate | 0,05 | 0,05 | 0,05 |
| Propylparaben | 0,20 | 0,20 | 0,20 |
| Dow Cominq 1403 Fluid | 1,00 | 1,00 | 1,00 |
| Dry Flo plus | 1,00 | 1,00 | 1,00 |
| TiO₂ | 0,50 | 0,50 | 0,50 |
| Hexandiol | 6,00 | 3,00 | |
| Propylenglycol | 5,00 | 5,00 | 5,00 |
| Glycerol | 5,00 | 3,00 | 3,00 |
| Methylparaben | 0,20 | 0,20 | 0,20 |
| Sodium Carbomer | 0,40 | 0,40 | 0,40 |
| Algae Extract | 1,00 | - | - |
| Hydrovance | 4,30 | 2,50 | 8,60 |
| Taurin | 1,00 | 1,00 | 0,30 |
| Perfume | 0,10 | 0,10 | 0,10 |
| Aqua | ad 100 | ad 100 | ad 100 |

### 2. Beispielserie: nicht erfindungsgemäß

| | 1 | 2 | 3 |
|---|---|---|---|
| Cetearyl Isononanoate | 4,00 | 4,00 | 4,00 |
| Mineral oil | 6,00 | 6,00 | 6,00 |
| Cutina CBS | 2,00 | 2,00 | 2,00 |
| Palmitic Acid / Stearic Acid | 1,50 | 1,50 | 1,50 |
| Ceteareth-30 | 1,00 | 1,00 | 1,00 |
| Dow Corning 200 Fluid 5 cst | 1,00 | 1,00 | 1,00 |
| Tocopheryl Acetate | 0,50 | 0,50 | 0,50 |
| Propylparaben | 0,30 | 0,30 | 0,30 |
| Pemulen TR 1 | 0,27 | 0,27 | 0,27 |
| Glycerin | 5,00 | 3,00 | 3,00 |
| Milchsäure | 0,26 | 0,26 | 0,26 |
| Propylenglycol | 5,00 | 5,00 | 5,00 |
| Methylparaben | 0,30 | 0,30 | 0,30 |
| Phenoxyethanol | 0,90 | 0,90 | 0,90 |
| Panthenol | 0,50 | - | - |
| Laminaria Digitata Extract | 1,00 | - | - |
| Natriumchlorid | 0,05 | 0,05 | 0,05 |
| Sepigel 305 | 0,50 | 0,50 | 0,50 |
| Silk Protein | 0,25 | 0,25 | 0,25 |
| Taurin | 1,00 | 0,50 | 2,00 |
| Calmosensine | 1,00 | 2,00 | - |
| Xanthan Gum | 0,20 | 0,20 | 0,20 |
| (2-Hydroxyethyl)harnstoff | 4,30 | 2,50 | 8,60 |
| Perfume | 0,30 | 0,30 | 0,30 |
| Silymarin Phytosome | 1,00 | - | - |
| Madecassoside | - | 1,00 | - |
| Calmsphere | - | - | 1,00 |
| Aqua | ad 100 | ad 100 | ad 100 |

### 3. Beispielserie: nicht erfindungsgemäß

| | 1 | 2 | 3 |
|---|---|---|---|
| Hydrogenated Lecithin | 0,50 | 0,50 | 0,50 |
| Isopropylstearate | 4,00 | 4,00 | 4,00 |
| Dibutyl Adipate | 2,00 | 2,00 | 2,00 |
| Tocopheryl Acetate | 0,50 | 0,50 | 0,50 |
| Cutina MD | 1,00 | 1,00 | 1,00 |
| Behenyl Alcohol | 2,00 | 2,00 | 2,00 |
| Dow Corning 200 Fluid 5 cst | 0,50 | 0,50 | 0,50 |
| Propylparaben | 0,20 | 0,20 | 0,20 |
| Dow Corning 9040 | 1,00 | 1,00 | 1,00 |
| Glycerol | 3,00 | 4,50 | 3,00 |
| Hexandiol | 6,00 | 3,00 | |
| Methylparaben | 0,20 | 0,20 | 0,20 |
| Sodium Carbomer | 0,30 | 0,30 | 0,30 |
| Algae Extract | 1,00 | - | - |
| Photosomes® | 0,20 | 0,20 | 0,20 |
| Dimethylmethoxy Chromanol | 0,01 | 0,01 | 0,01 |
| Propylenglycol | 5,00 | 5,00 | 5,00 |
| Matrixyl | 3,00 | - | - |
| Biopeptide CL | - | 2,00 | 2,00 |
| Matrixyl 3000 | - | 1,00 | 1,00 |
| Acnacidol PG | 1,00 | - | - |
| Seboregul 2 | | 1,50 | |
| Simulgel NS | 1,50 | 1,50 | 1,50 |
| TiO₂ | 0,50 | 0,50 | |
| Hydrovance | 2,50 | 4,30 | 8,60 |
| Taurin | 1,00 | 1,00 | 0,30 |
| Perfume | 0,35 | 0,35 | 0,35 |
| Aqua | ad 100 | ad 100 | ad 100 |

### 4. Beispielserie:

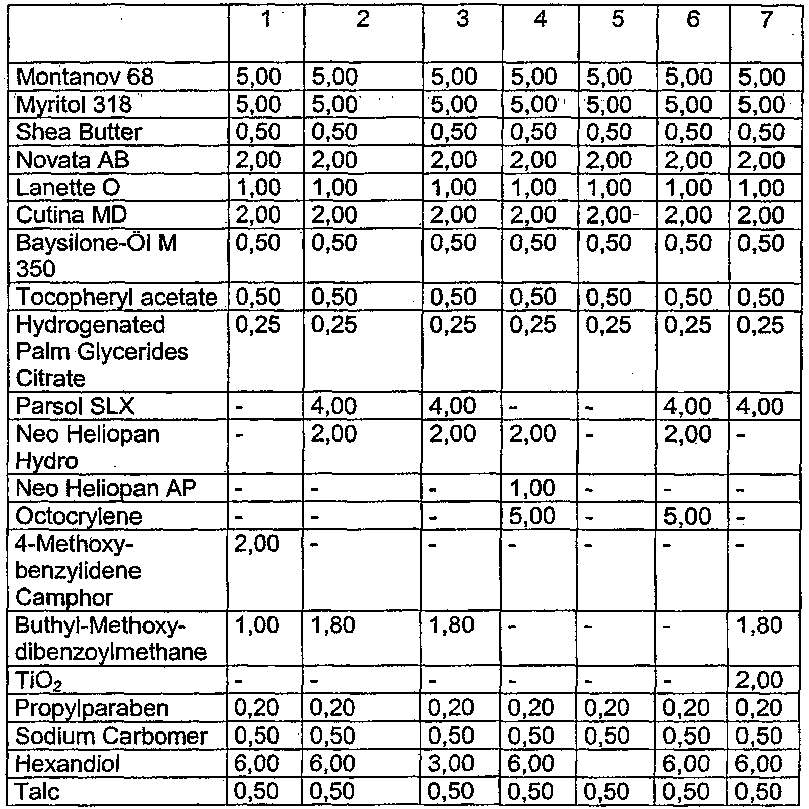

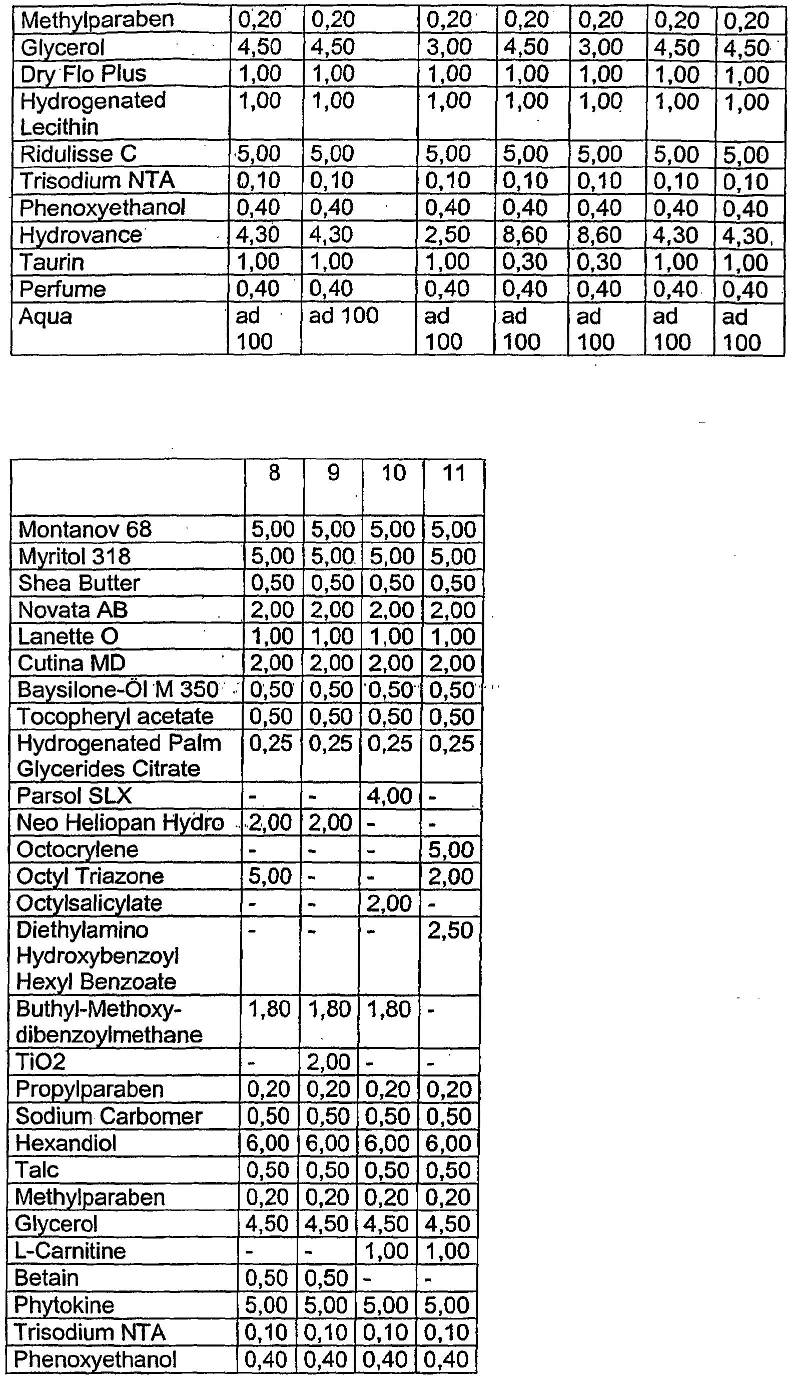

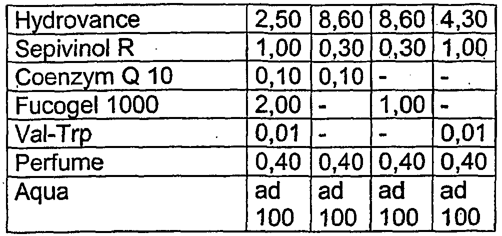

### 5. Beispielserie: nicht erfindungsgemäß

| | 1 | 2 | 3 |
|---|---|---|---|
| Montanov L | 3,00 | 3,00 | 3,00 |
| Dibutyl Adipate | 6,00 | 6,00 | 6,00 |
| Myritol 318 | 3,00 | 3,00 | 3,00 |
| Lanette O | 1,00 | 1,00 | 1,00 |
| Cutina MD | 0,50 | 0,50 | 0,50 |
| Baysilone-Öl M 350 | 0,50 | 0,50 | 0,50 |
| Dow Corning 9040 | 1,00 | 2,00 | - |
| Propylparaben | 0,20 | 0,20 | 0,20 |
| Cyclopentasiloxane | 1,50 | 1,50 | 1,50 |
| Glycerol | 5,00 | 5,00 | 5,00 |
| Sorbitol | 3,00 | 3,00 | 3,00 |
| Methylparaben | 0,20 | 0,20 | 0,20 |
| Dry Flo Plus | 0,50 | 0,50 | 0,50 |
| Sodium Carbomer | 0,10 | 0,10 | 0,10 |
| Laminata Digitata Extract | 0,50 | 0,50 | 0,50 |
| Hydrovance | 2,50 | 4,30 | 8,60 |
| Alpha-Glucosylrutin | 0,05 | - | 0,10 |
| Silymarine Phytosome | - | 0,05 | - |
| Sveltine LHYA923S | 2,00 | - | 3,00 |
| Dihydroxyaceton | - | 2,00 | - |
| allantoin | - | 0,50 | 0,50 |
| Perfume | 0,20 | 0,20 | 0,20 |
| Aqua | ad. 100 | ad. 100 | ad. 100 |

### 6. Wasser-in-Oel-Emulsion nicht erfindungsgemäß

| | 1 | 2 | 3 |
|---|---|---|---|
| Lameform TGI | 3,0 | 3,0 | 3,0 |
| Elfacos ST 9 | 0,5 | 0,5 | 0,5 |
| Microcrystalline Wax | 3,0 | 3,0 | 3,0 |
| Bis-Diglyceryl Polyacyladipate-2 | 1,0 | 1,0 | 1,0 |
| Paraffinöl | 8,0 | 8,0 | 8,0 |
| Vaseline | 2,0 | 2,0 | 2,0 |
| Vitamin-E-acetat | 2,0 | 2,0 | 2,0 |
| Methylparaben | 0,3 | 0,3 | 0,3 |
| Propylparaben | 0,3 | 0,3 | 0,3 |
| Isopropylisostearate | 8,0 | 8,0 | 8,0 |
| Glycerol | 5,0 | 3,0 | 3,0 |
| Mg-Sulfate | 0,5 | 0,5 | 0,5 |
| Hydrovance | 8,60 | 8,60 | 4,30 |
| Taurin | 0,30 | 1,00 | 1,00 |
| Milchsäure 80%ig | 0,560 | 0,560 | 0,560 |
| Algae Extract | 1,0 | - | - |
| Panthenol | 0,5 | 1,0 | 0,5 |
| Calendula Officinalis Flower Extract | 0,3 | - | - |
| Propylene Glycol | 0,5 | - | - |
| Parfum | 0,2 | 0,2 | 0,2 |
| Aqua | ad.100 | ad.100 | ad.100 |

### 7. Wasser-in-Silicon-Emulsion nicht erfindungsgemäß

| | 1 | 2 | 3 | 4 | 5 | 6 |
|---|---|---|---|---|---|---|
| Dimethicone | 2,50 | 2,50 | 2,50 | 2,50 | 2,50 | 2,50 |
| Cyclomethicone | 25,00 | 25,00 | 25,00 | 25,00 | 25,00 | 25,00 |
| Cetyl Dimethicone Copolyol | 2,00 | 2,00 | 2,00 | 2,00 | 2,00 | 2,00 |
| Sodium Chloride | 2,00 | 2,00 | 2,00 | 2,00 | 2,00 | 2,00 |
| Perfume | 0,30 | 0,30 | 0,30 | 0,30 | 0,30 | 0,30 |
| Methylparaben | 0,20 | 0,20 | 0,20 | 0,20 | 0,20 | 0,20 |
| Propylparaben | 0,20 | 0,20 | 0,20 | 0,20 | 0,20 | 0,20 |
| Phenoxyethanol | 0,40 | 0,40 | 0,40 | 0,40 | 0,40 | 0,40 |
| Glycerol | 5,00 | 2,50 | 5,00 | 5,00 | 2,50 | 5,00 |
| (2-Hydroxyethyl)urea | 4,30 | 8,60 | 2,50 | - | - | - |
| Hydrovance | - | - | - | 4,30 | 8,60 | 2,50 |
| Taurin | 1,00 | 1,00 | 0,30 | 1,00 | 1,00 | 0,30 |
| Na-Ascorbylphosphat | 1,00 | - | - | 1,00 | - | - |
| Ascorbylpalmitat | - | 1,00 | - | - | 1,00 | - |
| Vitamin B6 | 0,10 | 0,10 | 0,10 | 0,10 | 0,10 | 0,10 |
| Retinol | 0,10 | - | - | 0,10 | - | - |
| Alpha-Liponsäure | - | - | 0,10 | - | - | 0,10 |
| Coenzym Q 10 | - | 0,20 | - | - | 0,20 | - |
| Ectoin | 0,10 | 0,10 | 0,10 | 0,10 | 0,10 | 0,10 |
| Alpha-Bisabolol | 0,50 | 0,50 | - | 0,50 | 0,50 | - |
| Crodarom Chardonnay L | 2,00 | - | 1,00 | 2,00 | - | 1,00 |
| Soy Phytochemicals Concentrate SPC | - | 2,00 | - | - | 2,00 | - |
| Ultrasomes | 2,00 | 2,00 | 2,00 | 2,00 | 2,00 | 2,00 |
| Aqua | ad. 100 | ad. 100 | ad. 100 | ad. 100 | ad. 100 | ad. 100 |

### 3. Reinigungszubereitungen

### 1. Beispielserie: nicht erfindungsgemäß

| | 1 | 2 | 3 | 4 | 5 | 6 |
|---|---|---|---|---|---|---|
| Dipropylenglycol | 10,00 | 10,00 | 10,00 | 10,00 | 10,00 | 10,00 |
| Chlorhexidindigluconate | 1,00 | 1,00 | 1,00 | 1,00 | 1,00 | 1,00 |
| Poloxamer-184 | 3,00 | 3,00 | 3,00 | 3,00 | 3,00 | 3,00 |
| Panthenol | 0,50 | 0,50 | 0,50 | 0,50 | 0,50 | 0,50 |
| Chitosan Glycolate | 3,00 | 3,00 | 3,00 | 3,00 | 3,00 | 3,00 |
| PEG-40 Hydrogenated Castor Oil / Trideceth 9 / Propylene Glycol | 0,50 | 0,50 | 0,50 | 0,50 | 0,50 | 0,50 |
| Chlorella Vulgaris Extract | 0,50 | 0,50 | 0,50 | 0,50 | 0,50 | 0,50 |
| (2-Hydroxyethyl)urea | 2,50 | 4,30 | 8,60 | - | - | - |
| Hydrovance | - | - | - | 2,50 | 4,30 | 8,60 |
| Taurine | 1,00 | 0,50 | 0,30 | 1,00 | 0,50 | 0,30 |
| Perfume | 0,20 | 0,20 | 0,20 | 0,20 | 0,20 | 0,20 |
| Salicylsäure | 0,50 | - | - | 0,50 | - | - |
| Niacinamid | 0,50 | 0,50 | 0,50 | 0,50 | 0,50 | 0,50 |
| Vitamin B6 | 0,10 | 0,10 | 0,10 | 0,10 | 0,10 | 0,10 |
| Aqua | ad 100 | ad 100 | ad 100 | ad 100 | ad 100 | ad 100 |

### 2. Beispielserie: nicht erfindungsgemäß

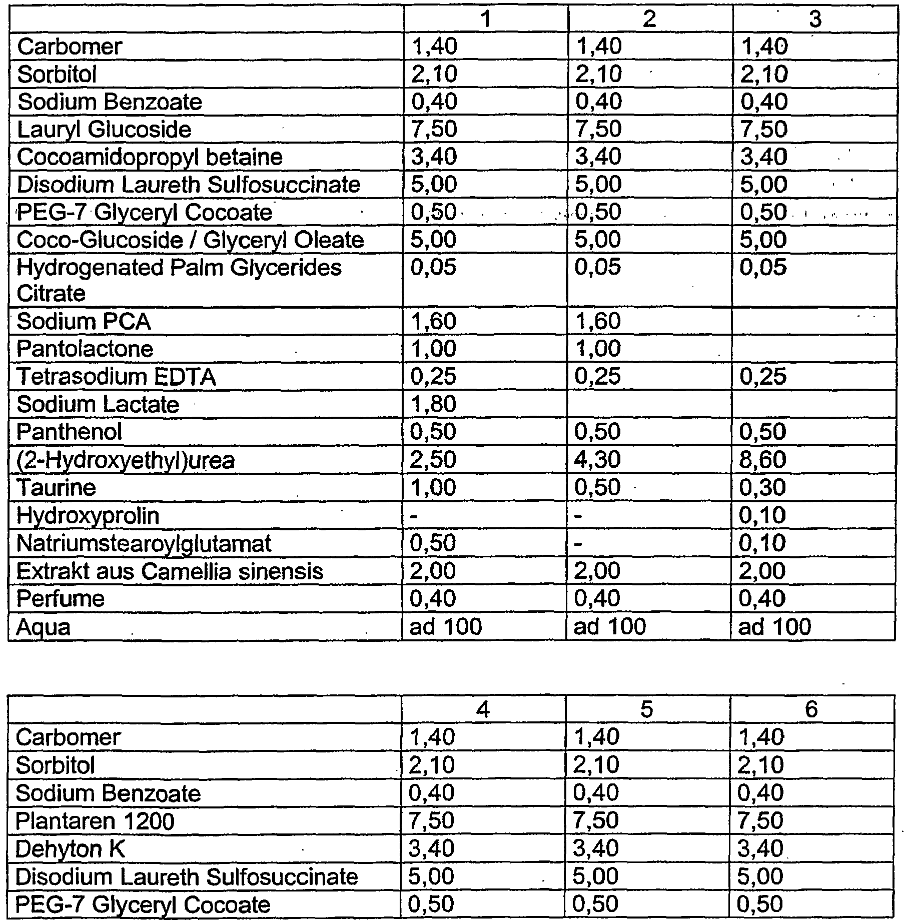

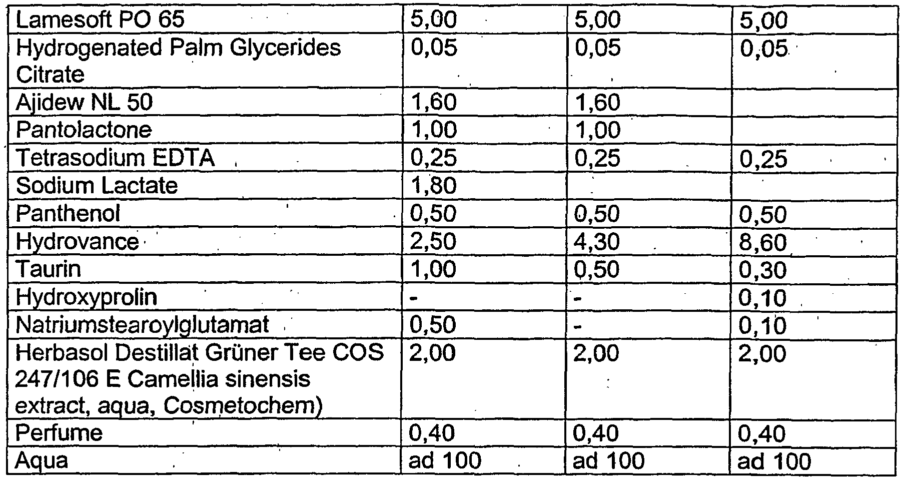

### Tagescremes

### 8. Beispielserie: nicht erfindungsgemäß

| | 1 | 2 | 3 |
|---|---|---|---|
| Carthamus Tinctorius | 3,00 | 3,00 | 3,00 |
| Caprylic/Capric Triglyceride | 5,00 | 5,00 | 5,00 |
| Cocoglycerides | 2,00 | 2,00 | 2,00 |
| Behenyl Alcohol | 1,00 | 1,00 | 1,00 |
| Glyceryl Stearate | 2,00 | 2,00 | 2,00 |
| Cetearyl Alcohol | 1,00 | 1,00 | 1,00 |
| Isopropylstearate | 4,00 | 4,00 | 4,00 |
| Shea Butter | 2,00 | 2,00 | 2,00 |
| Dimethicone | 1,00 | 1,00 | 1,00 |
| Hydrogenated Palm Glycerides Citrate | 0,05 | 0,05 | 0,05 |
| Propylparaben | 0,20 | 0,20 | 0,20 |
| Cyclopentasiloxane/Dimethiconol | 1,00 | 1,00 | 1,00 |
| Aluminium Starch Octenylsuccinate | 1,00 | 1,00 | 1,00 |
| TiO2 | 0,50 | 0,50 | 0,50 |
| Hexandiol | 6,00 | 3,00 | |
| Propylenglycol | 5,00 | 5,00 | 5,00 |
| Glycerol | 5,00 | 3,00 | 3,00 |
| Methylparaben | 0,20 | 0,20 | 0,20 |
| Sodium Carbomer | 0,40 | 0,40 | 0,40 |
| Algae Extract | 1,00 | | |
| (2-Hydroxyethyl)urea_{.} | 4,30 | 2,50 | 8,60 |
| Taurine | 1,00 | 1,00 | 0,30 |
| Perfume | 0,10 | 0,10 | 0,10 |
| Aqua | ad. 100 | ad. 100 | ad. 100 |

9. Beispielserie: nicht erfindungsgemäß

| | 1 | 2 | 3 |
|---|---|---|---|
| Cetearyl Isononanoate | 4,00 | 4,00 | 4,00 |
| Mineral oil | 6,00 | 6,00 | 6,00 |
| Glyceryl Stearate / Cetearyl Alcohol / Cetyl Palmitate / Cocoglycerides | 2,00 | 2,00 | 2,00 |
| Palmitic Acid / Stearic Acid | 1,50 | 1,50 | 1,50 |
| Ceteareth-30 | 1,00 | 1,00 | 1,00 |
| Dimethicone | 1,00 | 1,00 | 1,00 |
| Tocoperyl Acetate | 0,50 | 0,50 | 0,50 |
| Propylparaben | 0,30 | 0,30 | 0,30 |
| Acrylates/C10-30 Alkyl Acrylate Crosspolymer | 0,27 | 0,27 | 0,27 |
| Glycerol | 5,00 | 3,00 | 3,00 |
| Lactic Acid | 0,26 | 0,26 | 0,26 |
| Propylenglycol | 5,00 | 5,00 | 5,00 |
| Methylparaben | 0,30 | 0,30 | 0,30 |
| Phenoxyethanol | 0,90 | 0,90 | 0,90 |
| Panthenol | 0,50 | | |
| Laminaria Diqitata Extract | 1,00 | | |
| Sodium Chloride | 0,05 | 0,05 | 0,05 |
| Polyacrylamide / C13-14 Isoparaffin/Laureth-7 | 0,50 | 0,50 | 0,50 |
| Silk Protein | 0,25 | 0,25 | 0,25 |
| Xanthan Gum | 0,20 | 0,20 | 0,20 |
| (2-Hydroxyethyl)urea | 4,30 | 2,50 | 8,60 |
| Taurine | 1,00 | 1,00 | 0,30 |
| Perfume | 0,30 | 0,30 | 0,30 |
| Aqua | ad. 100 | ad. 100 | ad. 100 |

### 10. Beispielserie: nicht erfindungsgemäß

| | 1 | 2 | 3 |
|---|---|---|---|
| Hydrogenated Lecithin | 0,50 | 0,50 | 0,50 |
| Isopropylstearate | 4,00 | 4,00 | 4,00 |
| Dibutyl Adipate | 2,00 | 2,00 | 2,00 |
| Tocopheryl Acetate | 0,50 | 0,50 | 0,50 |
| Glyceryl Stearate | 1,00 | 1,00 | 1,00 |
| Behenyl Alcohol | 2,00 | 2,00 | 2,00 |
| Dimethicone | 0,50 | 0,50 | 0,50 |
| Propylparaben | 0,20 | 0,20 | 0,20 |
| Cyclomethicone/ Dimethicone Crosspolymer | 1,00 | 1,00 | 1,00 |
| Glycerol | 3,00 | 4,50 | 3,00 |
| Hexandiole | 6,00 | 3,00 | |
| Methylparaben | 0,20 | 0,20 | 0,20 |
| Sodium Carbomer | 0,30 | 0,30 | 0,30 |
| Algae Extract | 1,00 | | |
| Plancton Extract/Photosomes® | 0,20 | 0,20 | 0,20 |
| Dimethylmethoxy Chromanol | 0,01 | 0,01 | 0,01 |
| Propylenglycol | 5,00 | 5,00 | 5,00 |
| Palmitoyl Pentapeptide-3 | 3,00 | | |
| Palmitoyl Oligopeptide | | 2,00 | 2,00 |
| Palmitoyl Tetrapeptide-1 | | 1,00 | 1,00 |
| 10-Hydroxydecanoic acid/Acnacidol | 1,00 | | |
| Spiraea Ulmaria/Seburegul | | 1,50 | |
| Hydroxyethyl Acrylate / Sodium Acryloyldimethyl Taurate Copolymer / Squalane / Polysorbate 60 | 1,50 | 1,50 | 1,50 |
| TiO2 | 0,50 | 0,50 | |
| (2-Hydroxyethyl)urea | 2,50 | 4,30 | 8,60 |
| Taurine | 1,00 | 1,00 | 0,30 |
| Perfume | 0,35 | 0,35 | 0,35 |
| Aqua | ad. 100 | ad. 100 | ad. 100 |

### 11. Beispielserie:

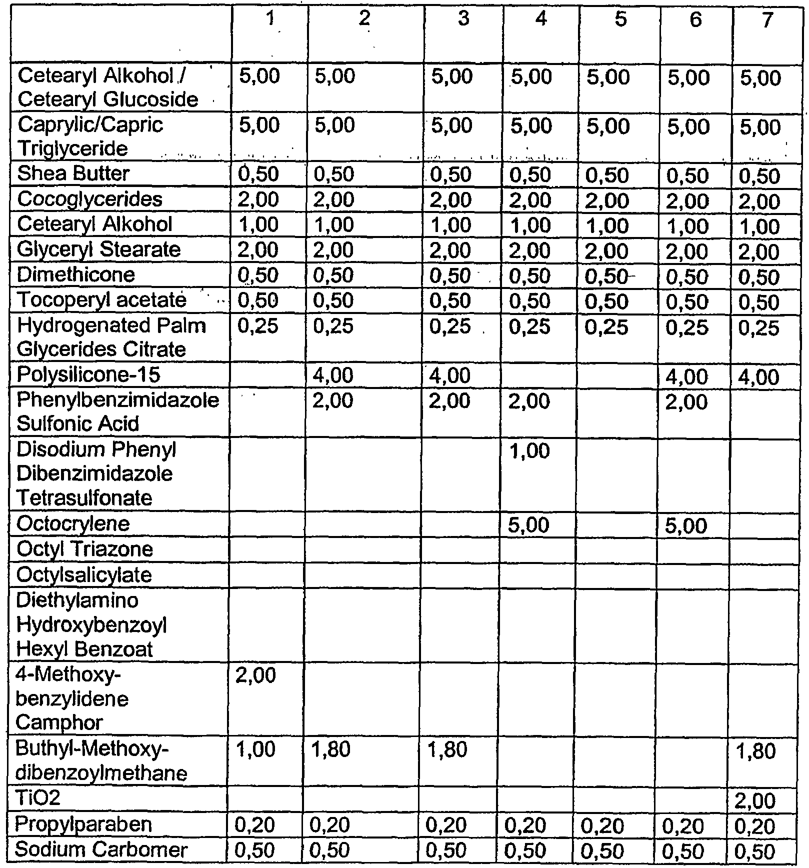

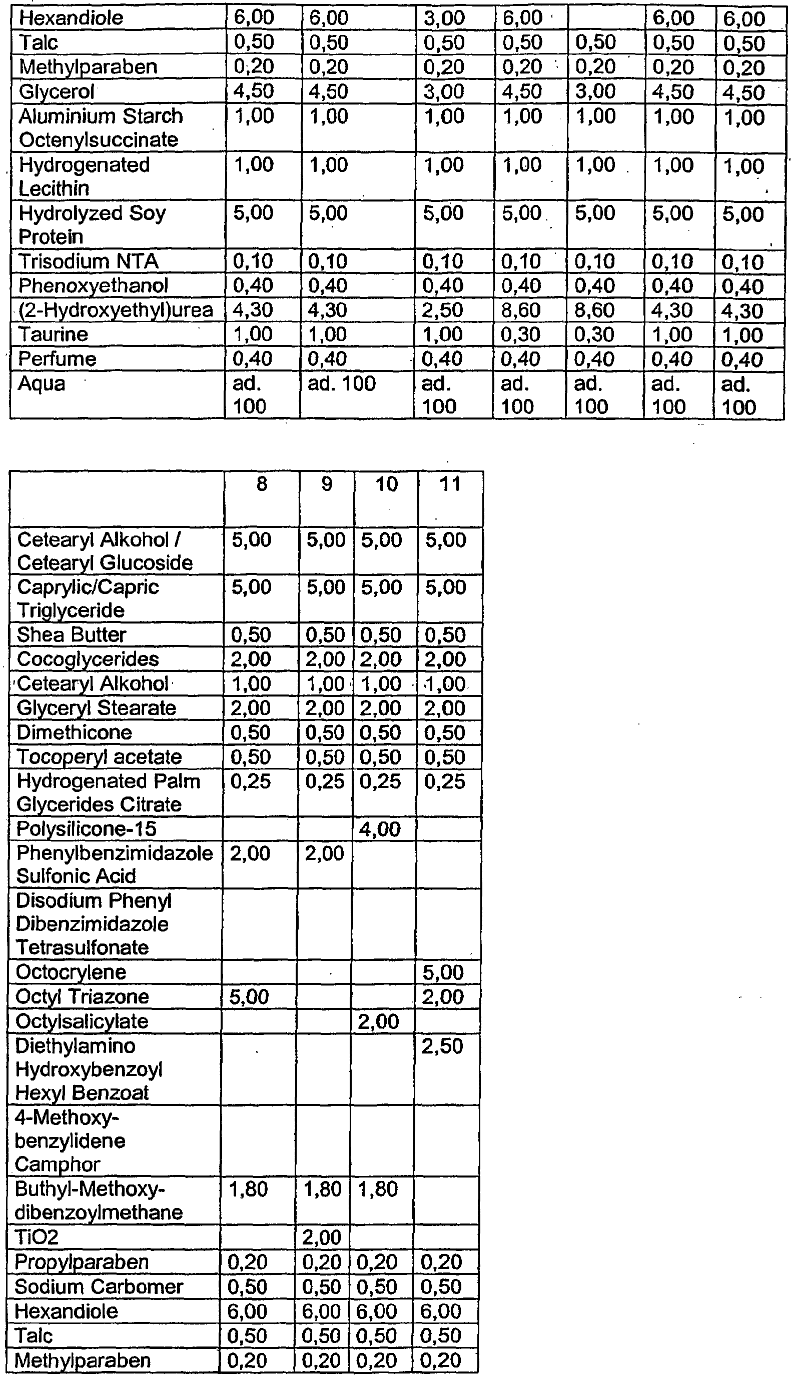

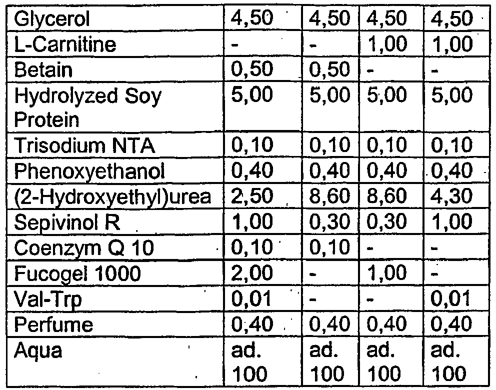

### 12. Beispielserie: nicht erfindungsgemäß

| | 1 | 2 | 3 |
|---|---|---|---|
| C14-22 Alcohols/C12-20 Alkyl Glucosid | 3,00 | 3,00 | 3,00 |
| Dibutyl Adipate | 6,00 | 6,00 | 6,00 |
| Caprylic/Capric Triglycerid | 3,00 | 3,00 | 3,00 |
| Cetearyl Alkohol | 1,00 | 1,00 | 1,00 |
| Glyceryl Stearate | 0,50 | 0,50 | 0,50 |
| Dimethicon | 0,50 | 0,50 | 0,50 |
| Dow Corning 9040 | 1,00 | 2,00 | - |
| Propylparaben | 0,20 | 0,20 | 0,20 |
| Cyclopentasiloxan | 1,50 | 1,50 | 1,50 |
| Glycerol | 5,00 | 5,00 | 5,00 |
| Sorbitol | 3,00 | 3,00 | 3,00 |
| Methylparaben | 0,20 | 0,20 | 0,20 |
| Aluminium Starch Octenylsuccinate | 0,50 | 0,50 | 0,50 |
| Sodium Carbomer | 0,10 | 0,10 | 0,10 |
| Laminata Diqitata Extract | 0,50 | 0,50 | 0,50 |
| (2-Hydroxyethyl)urea | 2,50 | 4,30 | 8,60 |
| Alpha-Glucosylrutin | 0,05 | - | 0,10 |
| Silymarin | - | 0,05 | - |
| Sveltine | 2,00 | - | 3,00 |
| Dihydroxyaceton | - | 2,00 | - |
| Allantoin | - | 0,50 | 0,50 |
| Perfume | 0,20 | 0,20 | 0,20 |
| Aqua | ad. 100 | ad. 100 | ad. 100 |

### 13. Wasser-in-Oel-Emulsion nicht erfindungsgemäß

| | 1 | 2 | 3 |
|---|---|---|---|
| Polyglyceryl-3 Diisostearate | 3,0 | 3,0 | 3,0 |
| PEG-45/Dodecyl Glycol Copolymer | 0,5 | 0,5 | 0,5 |
| Microcrystalline Wax | 3,0 | 3,0 | 3,0 |
| Bis-Diglyceryl Polyacyladipate-2 | 1,0 | 1,0 | 1,0 |
| Paraffinöl | 8,0 | 8,0 | 8,0 |
| Vaseline | 2,0 | 2,0 | 2,0 |
| Vitamin-E-acetat | 2,0 | 2,0 | 2,0 |
| Methylparaben | 0,3 | 0,3 | 0,3 |
| Propylparaben | 0,3 | 0,3 | 0,3 |
| Isopropylisostearate | 8,0 | 8,0 | 8,0 |
| Glycerol | 5,0 | 3,0 | 3,0 |
| Mg-Sulfate | 0,5 | 0,5 | 0,5 |
| (2-Hydroxyethyl)urea | 8,60 | 8,60 | 4,30 |
| Taurine | 0,30 | 1,00 | 1,00 |
| Milchsäure 80%ig | 0,560 | 0,560 | 0,560 |
| Algae Extract | 1,0 | | |
| Panthenol | 0,5 | 1,0 | 0,5 |
| Calendula Officinalis Flower Extract | 0,3 | | |
| Propylene Glycol | 0,5 | | |
| Parfum | 0,2 | 0,2 | 0,2 |
| Aqua | ad.100 | ad.100 | ad.100 |

### 14. Beispielserie: nicht erfindungsgemäß

| | 1 | 2 | 3 |
|---|---|---|---|
| C14-22 Alcohols/C12-20 Alkyl Glucosid | 3,00 | 3,00 | 3,00 |
| Dibutyl Adipate | 6,00 | 6,00 | 6,00 |
| Caprylic/Capric Triglycerid | 3,00 | 3,00 | 3,00 |
| Cetearyl Alkohol | 1,00 | 1,00 | 1,00 |
| Glyceryl Stearate | 0,50 | 0,50 | 0,50 |
| Dow Corning 200 Fluid, 0,65 cst | 0,50 | 0,50 | 0,50 |
| Dow Cominq 9040 | 1,00 | 2,00 | - |
| Propylparaben | 0,20 | 0,20 | 0,20 |
| Cyclopentasiloxan | 1,50 | 1,50 | 1,50 |
| Glycerol | 5,00 | 5,00 | 5,00 |
| Sorbitol | 3,00 | 3,00 | 3,00 |
| Methylparaben | 0,20 | 0,20 | 0,20 |
| Aluminium Starch Octenylsuccinate | 0,50 | 0,50 | 0,50 |
| Sodium Carbomer | 0,10 | 0,10 | 0,10 |
| Laminata Digitata Extract | 0,50 | 0,50 | 0,50 |
| (2-Hydroxyethyl)urea | 2,50 | 4,30 | 8,60 |
| Alpha-Glucosylrutin | 0,05 | - | 0,10 |
| Vegetal Filling Spheres | - | 1,00 | 2,00 |
| Sveltine | 2,00 | - | 3,00 |
| Dihydroxyaceton | - | 2,00 | - |
| Calmosensine | - | 0,50 | 1,00 |
| DSH-C N | 1,00 | - | 2,00 |
| Caomint | 3,00 | - | - |
| Ederline H | 0,50 | 1,00 | - |
| Omega CH-Aktivator | - | - | 2,00 |
| Perfume | 0,20 | 0,20 | 0,20 |
| Aqua | ad 100 | ad 100 | ad 100 |

### Liste der verwendeten Handelsprodukte

| Handelsname | INCI-Bezeichnung | Lieferant/Hersteller |
|---|---|---|
| | | |
| Acnacidol PG | Sebacic acid, 10-hydroxydecanoic acid, 1,10-decanediol | Vincience |
| Ajidew^{®} NL 50 | Sodium PCA | AJINOMOTO |
| Baysilone-Öl M 350 | Dimethicone | Bayer |
| Biopeptide CL | Glyceryl Polymethacrylate, Propylene Glycol, Palmitoyl Oligopeptide | Sederma |
| Calmosensine | Acetyl Dipeptide-1 Cetylester | Sederma |
| Caomint | Propylene Glycol, Aqua, Mentha piperita, Theobroma cacao | Solabia |
| Carbopol ETD 2020 | Acrylates/C10-30 Alkyl Acrylate Crosspolymer | Noveon |
| Cetiol^{®} HE | PEG-7 Glyceryl Cocoate | Cognis |
| Crodarom Chardonnay L | Propylene Glycol, Water, Vitis Vinifera (Grape) Seed Extract | Croda |
| Cutina CBS | Glyceryl Stearate / Cetearyl Alcohol / Cetyl Palmitate / Cocoglycerides | Cognis |
| Cutina MD | Glyceryl Stearate | Cognis |
| Dehyton^{®} K | Cocoamidopropyl Betaine (ca. 30 % Aktivsubstanz) | Cognis |
| Dow Corning 1403 Fluid | Cyclomethicone, Dimethiconol | Dow Corning |
| Dow Corning 200 Fluid (0,65 cSt) | Dimethicone | Dow Corning |
| Dow Corning 9040 | Cyclomethicone/ Dimethicone Crosspolymer | Dow Corning |
| Dow Corning^{®}200 Fluid, 5 cSt. | Dimethicone | Dow Corning |
| Dry Flo Plus | Aluminium Starch Octenylsuccinate | National Starch |
| DSH CN | Water, Dimethylsilanol Hyaluronate | Exsymol |
| Ederline H | PEG-40 Hydrogenated Castor Oil, PPG-2-Ceteareth-9, Pyrus Malus (Apple) Fruit Extract | Seporga |
| Ederline L | Hexyldecanol, Pyrus Malus (Apple) Fruit Extract | Seporga |
| Elfacos ST 9 | PEG-45/Dodecyl Glycol Copolymer | Akzo Nobel |
| EMULGADE^{®} SE | Glyceryl Stearate, Ceteareth-20, Ceteareth-12, Cetearyl Alcohol, Cetyl Palmitate | Cognis |
| Eumulgin B 3 | Ceteareth-30 | Cognis |
| Eusolex^{®} OCR | Octocrylene | Merck KGaA |
| Lameform TGI | Polyglyceryl-3 Diisostearate | Cognis |
| Lamesoft^{®} PO 65 | Coco-Glucoside, Glyceryl Oleate, Water | Cognis |
| LANETTE^{®} O | Cetearyl Alcohol | Cognis |
| Madecassoside | Centella Asiatica Extract | DSM |
| Matrixyl | Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Montanov 68 | Cetearyl Alkohol, Cetearyl Glucoside | Seppic |
| Montanov L | C14-22 Alcohols/C12-20 Alkyl Glucoside | Seppic |
| Myritol 318 | Caprylic/Capric Triglyceride | Cognis |
| Neo Heliopan AP | Disodium Phenyl Dibenzimidazole Tetrasulfonate | Symrise |
| Neo Heliopan Hydro | Phenylbenzimidazole sulfonic acid | Symrise |
| Novata AB | Cocoplycerides | Cognis |
| Omega-CH-Aktivator | Tripeptide-1 | GfN |
| Parsol SLX | Polysilicone-15 | DSM |
| Pemulen TR 1 | Acrylates/C10-30 Alkyl Acrylate Crosspolymer | |
| Photosomes | Aqua, Lecithin, Plancton Extract | AGI Dermatics |
| Phytokine | HYDROLYZED SOY PROTEIN | Coletica |
| Plantaren^{®} 1200 | Lauryl Glucoside, ca. 50 % Aktivsubstanz | Cognis |
| Ridulisse C | Hydrolyzed Soy Protein | Silab |
| Rooibos Herbasec MPE | Rooibos Extract | Cosmetochem |
| Seboregul | Butylene Glycol, Aqua, Spiraea Ulmaria | Silab |
| Sepigel^{®}305¹⁵ | Polyacrylamide, C₁₃-C₁₄ Isoparaffin, Laureth-7 | SEPPIC |
| Sepivinol R | Polyphenolreicher Extrakt aus Rotwein | Seppic |
| Silymarin Phytosome | Silybum Marianum Extract and Phospholipids | Indena SpA |
| Simulgel NS | Hydroxyethyl Acrylate / Sodium Acryloyldimethyl Taurate Copolymer / Squalane / Polysorbate 60 | Seppic |
| Soy Protein Isolate | | Protein Technology International |
| Sveltine LHYA923S | AQUA (WATER), BUTYLENE GLYCOL, CARNITINE, LECITHIN, CAFFEINE, CARBOMER, ESCULIN, CENTELLA ASIATICA EXTRACT, ATELOCOLLA-GEN, SODIUM CHONDROITIN SULFATE | Coletica |
| Texapon^{®} SB 3 KC | Disodium Laureth Sulfosuccinate, Aqua (ca. 38 - 42% Aktivsubstanz) | Cognis |
| Tioveil^{®}-AQ-N | Cl 77891 (Titanium Dioxide), Alumina, Silica, Sodium Polyacrylate | Uniqema (Tioxide Specialties) |
| Ultrasomes | Aqua, Lecithin, Micrococcus luteus extract | AGI Dermatics |
| Vegetal Filling Spheres | CAPRYLlC/CAPRIC TRIGLYCERIDE, SILICA DIMETHYL SILYLATE, HYDROLYZED WHEAT PROTEIN, BUTYLENE GLYCOL, PHENOXYETHA-NOL, METHYLPARABEN, ETHYLPARA-BEN, PROPYLPARABEN, BUTYLPARA-BEN, ISOBUTYLPARABEN | Coletica |

## Patentansprüche

1. Kosmetische oder dermatologische topische Zusammensetzungen, enthaltend in einem geeigneten kosmetischen oder dermatologischen Träger
(A) (2-Hydroxyethyl)-harnstoff sowie
(B) mindestens einen Wirkstoff, ausgewählt aus Sojaproteinhydrolysaten.

2. Verwendung einer Zusammensetzung gemäß Anspruch 1 zur nicht-therapeutischen, kosmetischen Behandlung und/oder Minimierung von trockener Haut.

## Claims

1. Cosmetic or dermatological topical compositions containing in a suitable cosmetic or dermatological carrier
(A) (2-hydroxyethyl) urea and
(B) at least one active ingredient selected from soya protein hydrolysates.

2. Use of a composition according to claim 1 for the non-therapeutic, cosmetic treatment and/or minimising of dry skin.

## Revendications

1. Compositions topiques, cosmétiques ou dermatologiques, contenant, dans un support cosmétique ou dermatologique approprié,
(A) de la (2-hydroxyéthyl)-urée ainsi que
(B) au moins un principe actif, choisi parmi les hydrolysats de protéines de soja.

2. Utilisation d'une composition selon la revendication 1 pour le traitement cosmétique, non thérapeutique et/ou la minimisation de la peau sèche.
